Europäisches Patentamt

(19) European Patent Office    (11) Veröffentlichungsnummer: **0 212 370**

Office européen des brevets    **B1**

(12)    # EUROPÄISCHE PATENTSCHRIFT

(45) Veröffentlichungstag der Patentschrifft:
21.03.90

(21) Anmeldenummer: 86110522.9

(22) Anmeldetag: 30.07.86

(51) Int. Cl. ⁵: **A 61 B   5/08, A 61 B   5/04**

(54)    Verfahren und Gerät zur Atmungsüberwachung.

(30) Priorität: 21.08.85 CH 3588/85

(43) Veröffentlichungstag der Anmeldung:
04.03.87 Patentblatt 87/10

(45) Bekanntmachung des Hinweises auf die Patenterteilung:
21.03.90 Patentblatt 90/12

㊻ Bennante Vertragsstaaten:
CH DE FR GB IT LI NL

(56) Entgegenhaltungen:
EP-A-0 082 655
FR-A-2 192 790
FR-A-2 528 304
GB-A-2 060 892

MEDICAL & BIOLOGICAL ENGINEERING &
COMPUTING, Band 20, Nr. 3, Mai 1982, Seiten 293-298,
Stevenage, Herts, GB; A.J. WILSON et al.: "Methods
of filtering the heart-beat artefact from the breathing
waveform of infants obtained by impedance
pneumography"

(73) Patentinhaber: **KONTRON-HOLDING AG**
**Bernerstrasse Süd 169**
**CH-8048 Zürich (CH)**

(72) Erfinder: **Chaumet, Serge Georges Julien**
**76 Rue d'Assas**
**F-75006 Paris (FR)**

(74) Vertreter: **Körber, Wolfhart, Dr.**
**Patentanwälte Dipl.-Ing. H. Mitscherlich Dipl.-Ing. K.**
**Gunschmann Dr.rer.nat. W. Körber Dipl.Ing. J. Schmidt-**
**Evers Dipl.-Ing. W. Melzer Steinsdorfstrasse 10**
**D-8000 München 22 (DE)**

## Beschreibung

Die Erfindung betrifft ein Verfahren zur Überwachung von die Atmung eines Patienten kennzeichnenden Wellenformen und zur Detektion von Wellenformen mit Eigenschaften, die für einen Atemstillstand kennzeichnend sind, wobei das Verfahren die Verarbeitung eines ersten elektrischen Signals mit einer die Atmung des Patienten kennzeichnenden Wellenform und eines zweiten das Elektrokardiogramm des Patienten kennzeichnenden elektrischen Signals umfaßt. Weiter ist ein Gerät zur Durchführung dieses Verfahrens Gegenstand der Erfindung.

Bei Verfahren zur Überwachung von die Atmung des Patienten repräsentierenden Wellenformen werden die Zuverlässigkeit, mit der ein Atemstillstandsalarm ausgelöst wird, die Genauigkeit der Messung der Dauer der Atemstillstandszeiten sowie die Empfindlichkeit, mit der das Meßsystem einen Atemstillstand feststellt, durch Artefakte stark beeinträchtigt, die in dem die Atmung des Patienten kennzeichnenden Signal, das z. B. durch Messung der Änderung der transthorakalen Impedanz gewonnen wird, synchron mit der Herztätigkeit auftreten. In diesem Fall sind die Artefakte, die als kardiovaskuläre Artefakte bezeichnet werden, auf die Tatsache zurückzuführen, daß die Bewegung des Herzens und die Variation der Blutströmung eine Änderung der Impedanz des Brustkastens hervorrufen.

Wenn in der Welle des Atmungssignals die vorgenannten Artefakte nicht vorhanden wären, könnten Atemstillstände zumindest vom Grundsatz her durch Überwachung der Amplitude der Welle des Atmungssignals und durch Erfassung der Intervalle ermittelt werden, in denen die Amplitude unter einem vorbestimmten Schwellenwert liegt. In der Praxis ist ein solches Verfahren nicht zuverlässig, weil die Amplitude der kardiovaskulären Artefakte, die selbst im Fall eines Atemstillstands (ein Atemstillstand sollte im Prinzip durch eine Welle des Atmungssignals angezeigt werden, die flach verläuft und deren Amplitude praktisch gleich Null ist) die gewählte Detektionsschwelle sehr oft überschreiten und daher den Anschein einer normalen Atmung erwecken kann. Falls man die Detektionsschwelle niedrig wählt, läuft man Gefahr, daß Atemstillstände unbemerkt bleiben. Wenn man hingegen eine hohe Detektionsschwelle wählt, ist die Gefahr gegeben, daß falsche Atemstillstandsalarme ausgelöst werden, wenn das Atmungssignal zwar eine schwache Amplitude aufweist, diese jedoch noch nicht so klein ist, daß sie einem Atemstillstand entspricht. Da die Amplitude der kardiovaskulären Artefakte häufig ebenso groß oder sogar größer ist als die Amplitude eines schwachen Atmungssignals, ist es für den Benutzer eines solchen Verfahrens unmöglich, eine geeignete Detektionsschwelle zu wählen, die eine zuverlässige Erfassung von Atemstillständen und eine genaue Messung von deren Dauer erlaubt.

Fluktuationen der Grundlinie des Atmungssignals und andere Störsignale, die dem Atmungssignal häufig überlagert sind, machen es noch schwieriger, Atemstillstände zuverlässig zu erfassen. Um trotz des Vorhandenseins kardiovaskulärer Artefakte die Detektion von Atemstillstände zuverlässiger zu machen, wurden bereits folgende Verfahren vorgeschlagen:

Bei einem ersten bekannten Verfahren (französische Patentanmeldung 2 267 734) vergleicht man das Zeitintervall, das der Periode des Atmungssignals entspricht, mit dem Intervall zwischen aufeinanderfolgenden QRS-Komplexen des das Elektrokardiogramm des Patienten kennzeichnenden Signals. Wenn die Differenz zwischen diesen Intervallen während eines vorbestimmten Zeitintervalls unter einem vorbestimmten Schwellenwert bleibt, geht man davon aus, daß das scheinbar existierende Atmungssignal in Wirklichkeit ein kardiovaskulärer Artefakt ist.

Bei einem zweiten bekannten Verfahren (französische Patentanmeldung 2 192 790) mißt man die Phasendifferenz zwischen dem Atmungssignal und dem das Elektrokardiogramm des Patienten kennzeichnenden Signal. Wenn diese Differenz während eines vorbestimmten Zeitintervalls unter einem vorbestimmten Schwellenwert bleibt, geht man davon aus, daß das scheinbar existierende Atmungssignal in Wirklichkeit ein kardiovaskulärer Artefakt ist.

Die beiden erwähnten bekannten Verfahren haben folgende Nachteile:

Da die kardiovaskulären Artefakte häufig im Bereich der Detektionsgrenze liegen, täuscht ihre aleatorische Erfassung die Frequenz- oder Phasenvergleichseinrichtungen und erweckt den Anschein einer normalen Atmung, was zur Folge hat, daß ein Atemstillstand nicht entdeckt wird.

Bei einem dritten bekannten Verfahren (britische Patentanmeldung 2 060 892) bildet man ein Signal, das der ersten zeitlichen Ableitung des Atmungssignals entspricht und prüft die Steilheit dieses Signals in Zeitintervallen, die den Intervallen zwischen aufeinanderfolgenden QRS-Komplexen des das Elektrokardiogramm des Patienten kennzeichnenden Signals entsprechen. Wenn diese Steilheit in dem Prüfintervall mehrere Male aufeinanderfolgend einen negativen Wert annimmt, nimmt man an, daß das scheinbar vorhandene Atmungssignal in Wirklichkeit ein kardiovaskulärer Artefakt ist.

Die Nachteile dieses dritten bekannten Verfahrens sind folgende:

- es ist von der Polarität des Signals abhängig und funktioniert nicht, wenn die Elektroden nicht korrekt angebracht sind,
- es ist gegen Geräusche jeder Art empfindlich. Inbesondere kann ein stark verrauschtes Zufallssignal als Atemstillstand gewertet werden, da ein solches mit großer Wahrscheinlichkeit negative Steilheiten innerhalb des Prüfintervalls aufweist,

- es trägt der Form des Artefakts im Zeitpunkt des QRS-Komplexes Rechnung, diese Form ist aber von einem Patienten zum anderen nicht identisch und sie ist von der Plazierung der Elektroden abhängig.

Dieses Verfahren kann daher bei bestimmten Personen unwirksam sein.

Der Erfindung liegt die Aufgabe zugrunde, ein Verfahren und ein Gerät zu dessen Durchführung zu schaffen, mit denen die Nachteile der oben erwähnten bekannten Verfahren und Geräte zumindest teilweise eliminiert werden können, so daß man zuverlässige Atemstillstandsalarmgaben erhält und trotz der Anwesenheit von kardiovaskulären Artefakten in dem Atmungssignal die Dauer jedes Atemstillstands mit größerer Präzision und Empfindlichkeit messen kann. Das Verfahren und das Gerät zu seiner Durchführung sollen außerdem so preiswert und raumsparend wie möglich sein, damit ihre Inkorporation in ein Atmungüberwachungssystem keine exzessive Verteuerung verursacht.

Erfindungsgemäß wird diese Aufgabe mit einem Verfahren der eingangs beschriebenen Art gelöst, das dadurch gekennzeichnet ist,

a) dass die Amplitude des ersten Signals, oder die Amplitude eines davon abgeleiteten Signals, im Zeitpunkt des Auftretens jedes QRS-Komplexes mit einem vorbestimmten Schwellenwert verglichen wird,

b) dass das erste Signal oder das davon abgeleitete Signal synchron mit dem Auftreten jedes QRS-Komplexes in dem zweiten Signal auf Null zurückgesetzt wird, und

c) dass ein einen Atemstillstand anzeigender Alarm ausgelöst wird, wenn die Amplituden des ersten Signals, oder des davon abgeleiteten Signals, in den genannten Zeitpunkten den Schwellenwert während eines vorbestimmten Zeitintervals nicht überschreiten.

Eine bevorzugte Ausführungsform des Verfahrens ist dadurch gekennzeichnet,

a) daß ein drittes Signal gebildet wird, das für die erste zeitliche Ableitung des ersten Signals kennzeichnend ist,

b) daß ein viertes Signal gebildet wird, das für das Integral des dritten Signals über erste Integrationsintervalle kennzeichnend ist, wobei jedes dieser Integrationsintervalle der Zeitspanne zwischen zwei aufeinanderfolgenden QRS-Komplexen des zweiten Signals entspricht,

c) dass die Amplitude, die das vierte Signal im Zeitpunkt des Auftretens jedes das Ende eines der Integrationsintervalle markierenden QRS-Komplexe annimmt, mit einem vorbestimmten Schwellenwert verglichen wird.

d) dass das vierte Signal synchron mit dem Auftreten jedes QRS-Komplexes in dem zweiten Signal auf Null zurückgesetzt wird, und

e) daß ein einen Atemstillstand anzeigender Alarm ausgelöst wird, wenn die Amplituden des vierten Signals in den genannten Zeitpunkten den Schwellenwert während eines vorbestimmten Zeitintervalls nicht überschreiten.

Gegenstand der Erfindung ist weiter ein Gerät zur Überwachung der Atmung eines Patienten mit Mitteln zur Bildung eines ersten elektrischen Signals mit einer Wellenform, die für die Atmung des Patienten kennzeichnend ist, und eines zweiten elektrischen Signals, das für das Elektrokardiogramm des Patienten kennzeichnend ist,

Ein solches Gerät ist erfindungsgemäß gekennzeichnet durch

a) Mittel zur Bildung eines dritten Signals, das für die erste zeitliche Ableitung des ersten Signal kennzeichnend ist,

b) Mittel zur Bildung eines vierten Signals, das für das Integral des dritten Signals über erste Integrationsintervalle kennzeichnend ist, wobei jedes dieser Integrationsintervalle dem Intervall zwischen zwei aufeinanderfolgenden QRS-Komplexen in dem zweiten Signal entspricht,

c) Mittel zum Vergleichen der Amplitude des vierten Signals in dem das Ende eines der genannten Integrationsintervalle markierenden Zeitpunkt des Auftretens jedes QRS-Komplexes mit einem vorbestimmten Schwellenwert.

d) Mittel zur Rücksetzung des vierten Signals auf Null synchron mit dem Auftreten jedes QRS-Komplexes in dem zweiten Signal, sowie

e) Mittel zur Auslösung eines einen Atemstillstand anzeigenden Alarms, wenn die Amplituden des vierten Signals in den genannten Zeitpunkten den Schwellenwert während eines vorbestimmten Zeitintervalls nicht überschreiten.

Die bei der Durchführung des Verfahrens gemäß der Erfindung und der Benutzung eines Geräts gemäß der Erfindung erzielbaren Vorteile sind

- eine beträchtliche Verbesserung der Zuverlässigkeit der Atemstillstandsalarmgabe,
- eine Vergrößerung der Empfindlichkeit des Meßsystems für die Erfassung von Atemstillständen,
- eine beträchtliche Erhöhung der Genauigkeit bei der Messung der Dauer jedes Atemstillstands sowie
- die Einfachheit und der niedrige Preis der für die Durchführung des Verfahrens erforderlichen Mittel bzw. für die Konstruktion des Geräts gemäß der Erfindung.

Im folgenden seien anhand der Zeichnungen Ausführungsbeispiele des Verfahrens und des Geräts gemäß der Erfindung näher erläutert:

Geräts gemäß der Erfindung näher erläutert:

Fig. 1 zeigt die Plazierung von Elektroden zur Gewinnung des Atmungssignals und des für das Elektrokardiogramm eines Patienten kennzeichnenden Signals,

Fig. 2 zeigt typische Wellenformen des Elektrokardiogramms und des Atmungssignals eines Patienten,

Fig. 3 veranschaulicht eine erste Ausführungsform des Verfahrens gemäß der Erfindung anhand einiger Wellenformen von dabei auftretenden Signalen für den Fall eines normalen Atmungssignals, d. h. eines Atmungssignals ohne Atemstillstand,

Fig. 4 zeigt eine bevorzugte Version der ersten Ausführungsform des Verfahrens gemäß der Erfindung für den Fall eines Atmungssignals, das keinen Atemstillstand aufweist, jedoch besonders ungünstig verläuft,

Fig. 5 veranschaulicht ein Verfahren gemäß der Erfindung, das dem anhand von Fig. 4 beschriebenen Verfahren analog ist, jedoch für den Fall eines Atemstillstands bei Anwesenheit kardiovaskulärer Artefakte,

Fig. 6 zeigt typische Wellenformen der kardiovaskulären Artefakte,

Fig. 7 zeigt eine erste Ausführungsform einer elektronischen Einrichtung zur Durchführung des Verfahrens gemäß der Erfindung,

Fig. 8 zeigt eine modifizierte Version der Einrichtung gemäß Fig. 7,

Fig. 9 zeigt eine zweite Ausführungsform der Einrichtungen gemäß Fig. 7 und 8,

Fig. 10 zeigt eine dritte Ausführungsform der Einrichtungen gemäß Fig. 7 und 8,

Fig. 11 zeigt typische Form der Amplituden-Frequenz-Charakteristik der Differenzierschaltung 72 in Fig. 9 bis 10 und 12,

Fig. 12 zeigt das Blockschaltbild eines Geräts mit der Einrichtung gemäß Fig. 9 für die Erfassung des Atemstillstands bei der Überwachung des Atmungssignals,

Fig. 13 veranschaulicht eine Ausführungsform des Verfahrens gemäß der Erfindung anhand einiger Wellenformen der beteiligten Signale,

Fig. 14 zeigt die schematische Darstellung einer Vorrichtung für Durchführung des in Fig. 13 veranschaulichten Verfahrens.

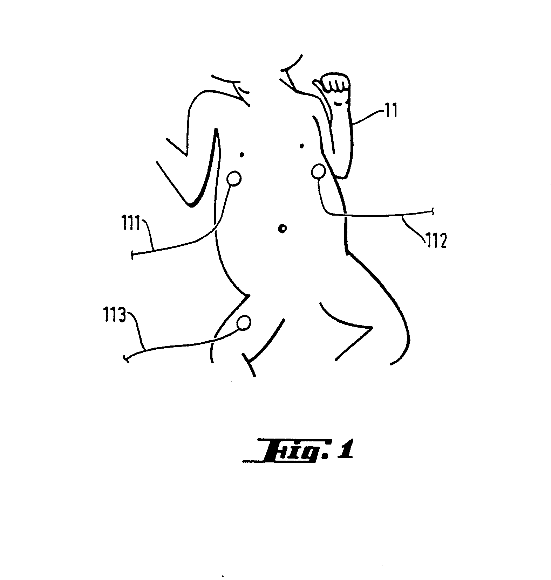

Fig. 1 zeigt die Plazierung von Elektroden 111 und 112 für die Gewinnung eines Signals, das für die Atmung kennzeichnend ist, sowie eines Signals, das für das Elektrokardiogramm eines Patienten z. B. eines Neugeborenen, kennzeichnend ist. Bei dem in Fig. 1 dargestellten Beispiel werden diese beiden Signale mit Hilfe desselben Elektrodensatzes gewonnen. Es ist im Rahmen der Erfindung jedoch auch möglich, jedes dieser Signale unabhängig von dem anderen mit getrennten Mitteln zu gewinnen.

Eine dritte Elektrode 113 kann fakultativ als Potentialreferenz verwendet werden.

Die in Fig. 1 gezeigten Elektroden ermöglichen die Gewinnung des Atmungssignals durch Messung der Thoraximpedanz. Dieses Signal kann auch durch andere Mittel gewonnen werden, z. B. einen Meßfühler für die Thoraxbeanspruchung, einen Verschiebungs- oder Beschleunigungsfühler, einen akustischen Aufnehmer oder einen pneumatischen Sensor für die Detektion der Druckveränderungen einer pneumatischen Matte usw.

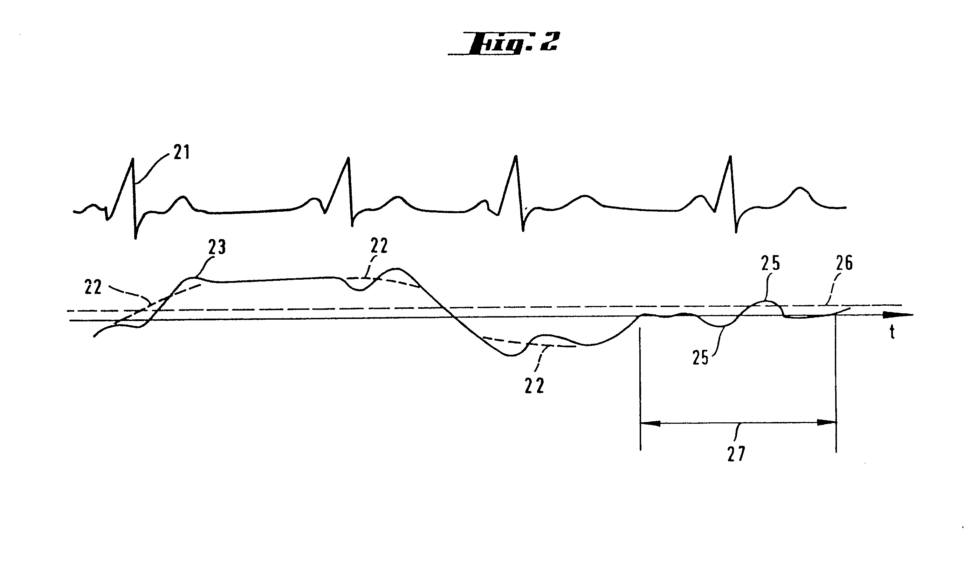

Fig. 2 zeigt typische Wellenformen der folgenden gleichzeitig gewonnenen Signale eines Patienten:

- eine Welle 21, die für das Elektrokardiogramm des Patienten 11 kennzeichnend ist,
- eine Welle 22, die die Atmung des Patienten für den Idealfall kennzeichnet, in dem diese Welle keine kardiovaskulären Artefakte beinhaltet, sowie
- eine Wellenform 23, die für das Atmungssignal kennzeichnend ist, wenn dieses kardiovaskuläre Artefakte aufweist.

In dem Zeitintervall 27 in Fig. 2 ist die Amplitude der Welle 22 des reinen Atmungssignals praktisch gleich Null, was einem Atemstillstand entspricht. Die Welle 23 besitzt hingegen eine Amplitude, die eine Detektionschwelle 26 überschreitet, weil sie einen kardiovaskulären Artefakt beinhaltet, der durch den Teil 25 der Welle 23 dargestellt ist. Dieser Artefakt verhindert also die Detektion des Atemstillstands mit Hilfe der Detektionsschwelle 26 und verleitet zu der Annahme, daß die Atmung in dem Intervall 27 normal sei. Falls man versucht, Atemstillstände durch Vergleich der Amplitude des Atmungssignals 23 mit einer Detektionsschwelle 26 zu erfassen, sieht man sich folgenden Schwierigkeiten gegenüber:

- falls die gewählte Detektionsschwelle niedrig ist, besteht die Gefahr, daß Atemstillstände nicht entdeckt werden (dies ist in dem Intervall 27 von Fig. 2 der Fall),
- wenn hingegen eine hohe Detektionsschwelle gewählt wird, läuft man Gefahr, im Fall eines normalen Atmungssignals, das jedoch eine schwache Amplitude besitzt, falsche Atemstillstandsalarme auszulösen.

In allen Fällen, d. h. unabhängig von der Größe der gewählten Detektionsschwelle, verursacht die Anwesenheit von kardiovaskulären Artefakten eine Ungenauigkeit bei der Messung der tatsächlichen Dauer der Atemstillstände (Apnoen), so daß es für den Benutzer schwierig ist, eine geeignete Detektionsschwelle 26 (durch Regelung der Empfindlichkeit) auszuwählen. Die Amplitude des Atmungssignals variiert nämlich nicht nur von Patient zu Patient, sie ändert sich außerdem zeitlich bei ein und demselben Patienten. Durch Anwendung der weiter unten beschrieben Verfahren gemäß der Erfindung lassen sich diese Schwie-

## 7

rigkeiten überwinden.

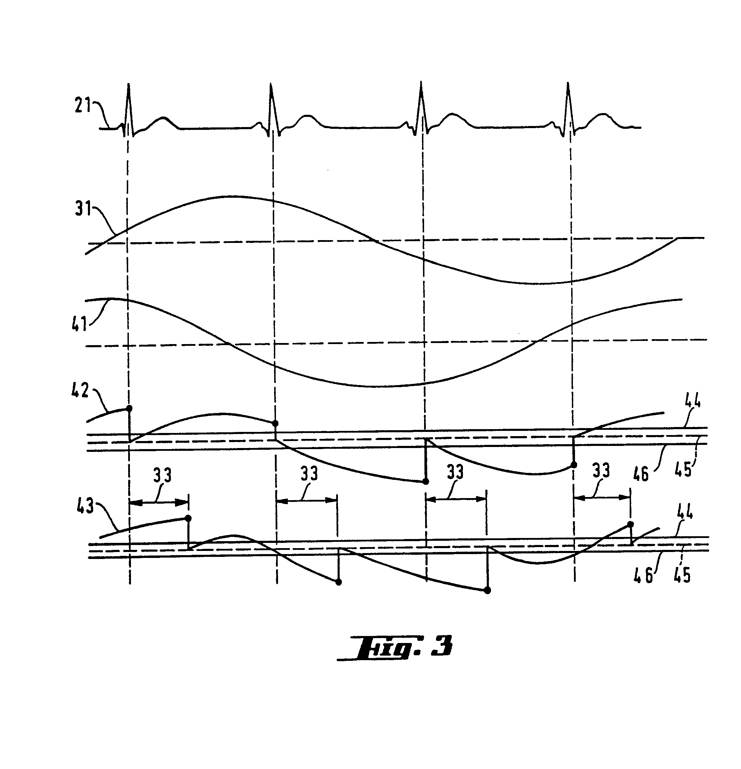

Eine erste Ausführungsform des Verfahrens gemäß der Erfindung für den Fall einer normalen Atmung ohne Atemstillstand wird durch die in Fig. 3 dargestellten Wellenformen veranschaulicht, in der die Zeitachsen durch unterbrochene horizontale Linien dargestellt sind. Dieses Verfahren geht aus von zwei gleichzeitig gewonnenen Signalen eines Patienten: Von dem Signal 21, das für das Elektrokardiogramm kennzeichnend ist, und dem Atmungssignal 31. Letzteres beinhaltet in der Praxis kardiovaskuläre Artefakte. Da diese Artefakte indessen auf das Resultat des Verfahrens für den in Fig. 3 dargestellten Fall keinen großen Einfluß haben, zeigt das Signal 31 zur Vereinfachung der Darstellung in Gig. 3 solche Artefakte nicht.

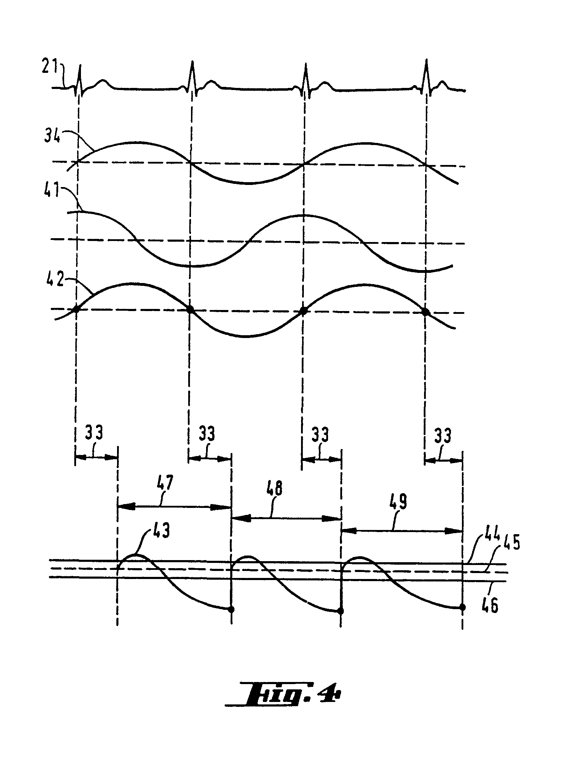

Bei dem Verfahren gemäß der Erfindung bildet man ein Signal 41, dessen Wellenform der ersten zeitlichen Ableitung der Wellenform des Atmungssignals 31 entspricht, sowie ein Signal 42, dessen Wellenform dem Integral des Signals 41 über Integrationsintervalle entspricht, die jeweils durch das Intervall zwischen zwei aufeinanderfolgenden QRS-Komplexen des Signals 21 gekennzeichnet sind, wobei beim Auftreten jedes QRS-Komplexes auf Null zurückgesetzt wird. In Fig. 3 und 4 ist das Auftreten dieser QRS-Komplexe durch unterbrochene vertikale Linien gestellt.

Die Amplitude, die das Signal 42 im Zeitpunkt des Auftretens jedes QRS-Komplexes annimmt, der das Ende eines der Integrationsintervalle markiert, wird mit einem Schwellenwert 44 für die Amplituden mit positiver Polarität und einem Schwellenwert 46 für die Amplituden mit negativer Polarität verglichen. Wenn die Amplituden des Signals 42 in den genannten Zeitpunkten den Schwellenwert 44 bzw. 46 innerhalb eines vorbestimmten Zeitintervalls nicht überschreiten, wird ein Alarm ausgelöst, der einen Atemstillstand anzeigt.

Das vorangehend beschriebene Verfahren besitzt folgende Vorteile:

— die Verwendung der ersten zeitlichen Ableitung (Bildung des Signals 41) einerseits und die Rücksetzung auf Null am Ende jedes Integrationsintervalls (bei der Bildung des Signals 42) andererseits bewirken, daß die Amplitude des Signals 42 von Fluktuationen des Grundlinie des Atmungssignals 31 nicht beeinflußt wird,
— die Integration des Signals 41 stellt zusätzlich eine Tiefpaßfilterung dar, durch die der Einfluß anderer (als der kardiovaskulären) Artefakte sowie Ungenauigkeiten in der Reproduzierbarkeit minimiert werden.

Das vorangehend beschriebene Verfahren ist zwar allgemein anwendbar, in dem in Fig. 4 dargestellten speziellen Fall, bei dem die Frequenz des Atmungssignals 34 exakt der halben Frequenz des EKG-Signals 21 entspricht und darüberhinaus das Signal 34 eine ungünstige Pha-

## 8

senbeziehung zu dem Signal 21 hat, ist jedoch das Verfahren unwirksam. In diesem speziellen Fall nimmt die Amplitude des Signals 42 am Ende jedes Integrationsintervalls stets den Wert Null an und erweckt den Anschein, daß ein Atemstillstand vorliegt. Um dieser Schwierigkeit zu begegnen, bildet man bei einer bevorzugten Ausführungsform des oben beschriebenen Verfahrens zusätzlich ein Signal 43, dessen Wellenform dem Integral des Signals 41 über die Integrationsintervalle 47, 48, 49 usw. entspricht, wobei jedes dieser Integrationsintervalle um eine Zeitspanne 33 von beispielsweise 200 ms gegenüber den denjenigen Integrationsintervallen versetzt ist, die für die Bildung des Signals 42 verwendet werden. Man vergleicht die Amplitude des Signals 43 in dem Zeitpunkt, der das Ende jedes dieser Integrationsintervalle 47, 48, 49 usw. markiert, mit den vorbestimmten Schwellenwerten 44 bzw. 46. Bei dieser bevorzugten Version der ersten Ausführungsform des Verfahrens gemäß der Erfindung wird der Atemstillstandsalarm nur dann ausgelöst, wenn die Amplituden des Signals 42 und die Amplituden des Signals 43 am Ende der betreffenden Integrationsintervalle den Schwellenwert während eines vorbestimmten Zeitintervalls von beispielsweise 3 Sekunden nicht überschreiten.

In dem besonders ungünstigen Fall von Fig. 4 überschreiten die Amplituden des Signals 43 am Ende der Integrationsintervalle den Schwellenwert 46 und verhindern dadurch mit Sicherheit die Auslösung eines falschen Atemstillstandsalarms.

In dem allgemeineren und häufiger auftretenden Fall, der in Fig. 3 dargestellt ist, überschreiten die Amplitudenwerte der Signale 42 und 43 am Ende der entsprechenden Integrationsintervalle die Schwellenwerte 44 bzw. 46 und verhindern sicher die Auslösung eines falschen Atemstillstandsalarms.

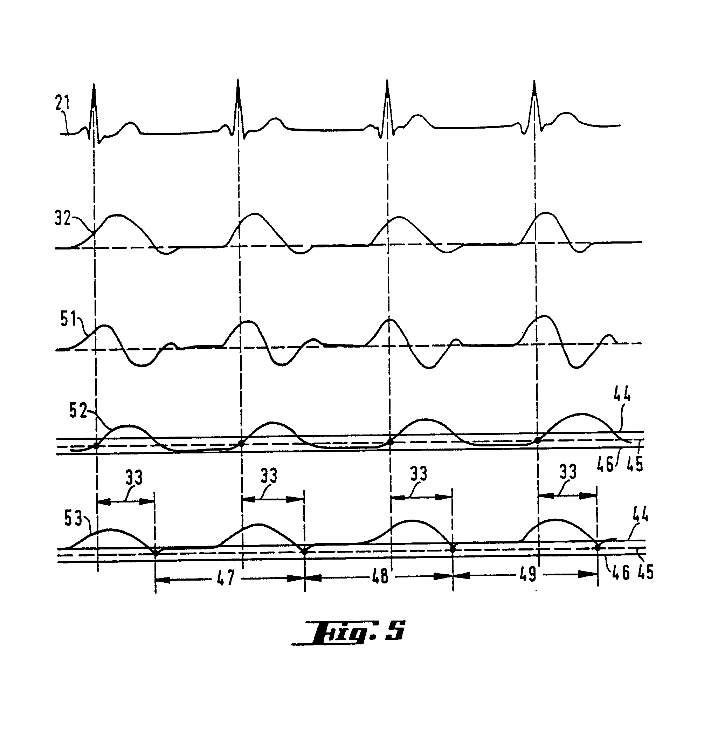

Im Fall eines Atemstillstands bei gleichzeitiger Anwesenheit von kardiovaskulären Artefakten gelten bei der ersten Ausführungsform des Verfahrens gemäß der Erfindung die in Fig. 5 dargestellten Wellenformen, wobei die Zeitachsen durch unterbrochene horizontale Linien angedeutet sind. Wie im oben beschriebenen Fall wird das Verfahren auf der Grundlage zweier Signale durchgeführt, die gleichzeitig von einem Patienten aufgenommen werden: des Signals 41, das das Elektrokardiogramm wiedergibt, und eines Signals 32, das mit den gleichen Mitteln gewonnen wird, die auch für die Gewinnung des Atmungssignals 31 in Fig. 3 verwendet werden. Da das Signal 32 einen Atemstillstand repräsentiert, zeigt seine Wellenform fast ausschließlich kardiovaskuläre Artefakte.

Bei dem Verfahren gemäß der Erfindung bildet man ein Signal 51, dessen Wellenform der ersten zeitlichen Ableitung der Wellenform des Signals 32 enspricht, sowie ein Signal 52, dessen Wellenform für das Integral des Signals 51 über Integrationsintervalle kennzeichnend ist, die jeweils durch die Zeitspanne zwischen aufeinanderfolgenden QRS-Komplexen des Signals 21 ist in Fig. 5 jeweils durch eine gestrichelte vertikale Linie angedeutet.

Die Amplitude des Signals 52 im Zeitpunkt des Auftretens der einzelnen QRS-Komplexe, die das Ende eines der Integrationsintervalle markieren, wird für Amplituden mit positiver Polarität mit dem Schwellenwert 44 und für Amplituden mir negativer Polarität mit dem Schwellenwert 46 verglichen.

Fig. 5 zeigt, daß die Amplituden des Signals 52 zu den oben definierten Zeitpunkten in einem Atemstillstandsintervall unter den Schwellenwerten 44 und 46 liegen. Bei der ersten Ausführungsform des Verfahrens gemäß der Erfindung wird ein Alarm ausgelöst und ein Atemstillstand angezeigt, wenn die genannten Amplituden für die Dauer eines vorbestimmten Zeitintervalls unter den genannten Schwellenwerten bleiben.

Zur Verbesserung der Zuverlässigkeit des Verfahrens gemäß der Erfindung bildet man bei einer bevorzugten Version zusätzlich ein Signal 53, dessen Wellenform das Integral des Signals 51 über Integrationsintervalle 47, 48, 49 usw. wiedergibt, wobei jedes dieser Integrationsintervalle gegenüber einem der für die Bildung des Signal 52 verwendeten Integrationsintervalle um das das Intervall 33 versetzt ist. Man vergleicht die Amplitude des Signals 53 in dem Zeitpunkt, der das Ende jedes dieser Integrationsintervalle 47, 48, 49 usw. markiert, mit den Schwellenwerten 44 bzw. 46. Fig. 5 zeigt, daß in einem Atemstillstandsintervall die Amplitude der Signale 52 und 53 am Ende der betreffenden Integrationsintervalle unterhalb der Schwellenwerte 44 bzw. Bei der bevorzugten Version der ersten Ausführungsform des Verfahrens gemäß der Erfindung löst man einen Atemstillstandsalarm aus, wenn die genannten Amplituden während eines vorbestimmten Zeitintervalls unter den genannten Schwellenwerten bleiben.

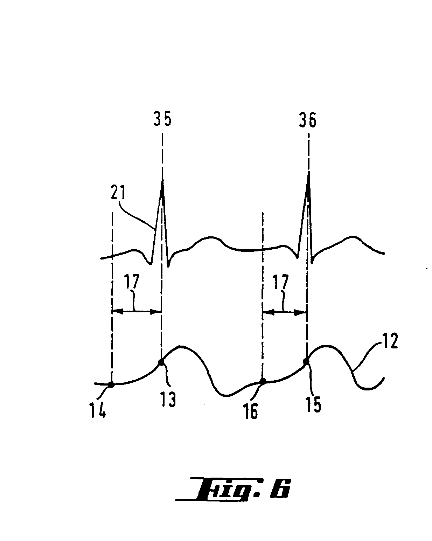

Die Wirksamkeit des Verfahrens gemäß der Erfindung beruht auf gewissen Eigentümlichkeiten der kardiovaskulären Artefakte. Wie aus Fig. 6 hervorgeht, kann die Wellenform 12 eines Signals, das für die kardiovaskulären Artefakte kennzeichnend ist, beliebige Form haben, es ist jedoch im allgemeinen hinreichend periodisch, mit dem für das Elektrokardiogramm kennzeichnenden Signal 21 synchron und wiederholt sich in seiner Form. Wie in Fig. 6 dargestellt ist, nimmt die Wellenform 12 des für einen kardiovaskulären Artefakt repräsentativen Signals für jeden Zeitpunkt seiner Periode wieder den gleichen Amplitudenwert an. So ist die Amplitude des Punktes 13 der Welle 12 beim Auftreten eines QRS-Komplexes des Signals 21 im Zeitpunkt 35 dieselbe wie diejenige des Punktes 15 im Zeitpunkt 36 beim Auftreten des nächstfolgenden QRS-Komplexes. Die Punkte 14 und 16, die den Punkten 13 bzw. 15 der Welle 12 vorangehen und von diesen durch Zeitintervalle 17 getrennt sind, haben ebenfalls dieselbe Amplitude. Diese zuletzt beschriebene Eigenschaft der Wellenform der kardiovaskulären Artefakte ist eine hinreichende Bedingung dafür, daß die Verfahren gemäß der Erfindung eine zuverlässige Erfassung der Atemstillstände und eine präzise Messung ihrer Dauer bei der Atmungsüberwachung eines Patienten erlauben.

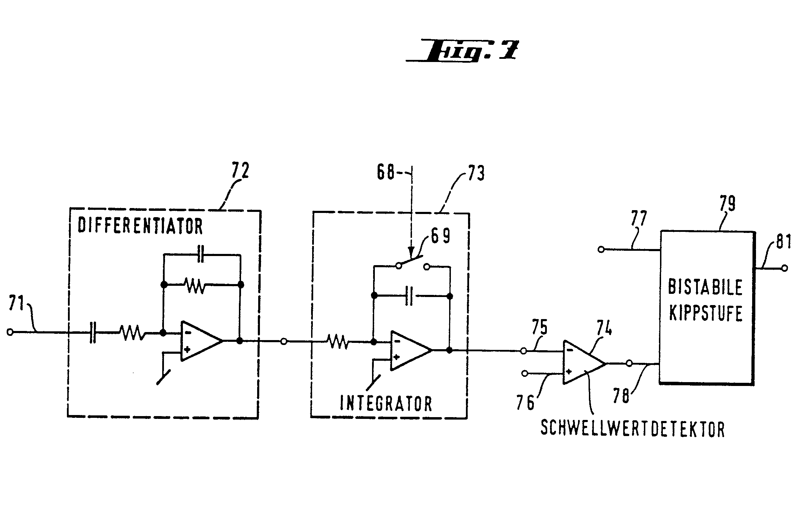

Fig. 7 zeigt ein erstes Ausführungsbeispiel einer elektronischen Einrichtung für die Durchführung eines Verfahrens gemäß der Erfindung. Diese Einrichtung umfaßt die Reihenanordnung folgender Schaltungen: einer Differenzierschaltung 72, einer Integrationsschaltung 73, eines Schwellwertdetektors 74 und einer bistabilen Kippstufe 79 in Form eines D-Flip-Flops.

Das Atmungssignal wird dem Eingang 71 der Differenzierschaltung 72 zugeführt. Diese liefert an ihrem Ausgang ein Signal, das für die erste zeitliche Ableitung des Atmungssignals repräsentativ ist. Dieses Signal wird dem Eingang des Integrators 73 zugeführt. Dieser liefert an seinem Ausgang ein Signal, das kennzeichnend ist für das Integral des seinem Eingang zugeführten Signals über Integrationsintervalle, die jeweils dem Intervall zwischen aufeinanderfolgenden QRS-Komplexen des Elektrokardiogramms des Patienten entsprechen. Der Integrator 73 wird beim Auftreten jedes QRS-Komplexes auf Null zurückgesetzt, indem ein Schalter 69 kurzzeitig geschlossen wird. Dieses Schalterschließen wird durch ein Signal gesteuert, das für den entsprechenden QRS-Komplex kennzeichnend ist und aus dem Signal des Elektrokardiogramms mit Hilfe einer geeigneten Detektorschaltung hergeleitet wird. In Fig. 7 ist das Schließen des Schalters 69 durch eine unterbrochene Linie 68 angedeutet. Das vom Ausgang des Integrators 73 gelieferte Signal wird einem ersten Eingang 75 des Schwellwertdetektors 74 zugeführt. Einem zweiten Ausgang 76 des Schwellwertdetektors 74 wird eine Spannung zugeführt, die einem vorbestimmten Schwellenwert entspricht. In Abhängigkeit von dem Ergebnis des Vergleichs der an seinen Eingängen anliegenden Signale liefert der Schwellwertdetektor 74 an seinem Ausgang ein Signal, das entweder dem logischen Zustand "1" oder "0" entspricht. Dieses Signal wird über die Leitung 18 dem D-Eingang der Kippstufe 79 zugeführt. Dieser Eingang der Kippstufe ist derjenige, der bei einer Verwendung in digitalen Schaltungen die Taktimpulse aufnimmt. Das Ausgangssignal der Kippstufe 79 wird an eine Leitung 81 abgegeben und einer (in Fig. 7 nicht dargestellten) Analysierschaltung zugeführt, die einen Atemstillstandsalarm auslöst, wenn das Ausgangssignal der Kippstufe 79 anzeigt, daß die Amplituden des Ausgangssignals des Integrators während eines vorbestimmten Zeitintervalls den Schwellenwert nicht überschreiten.

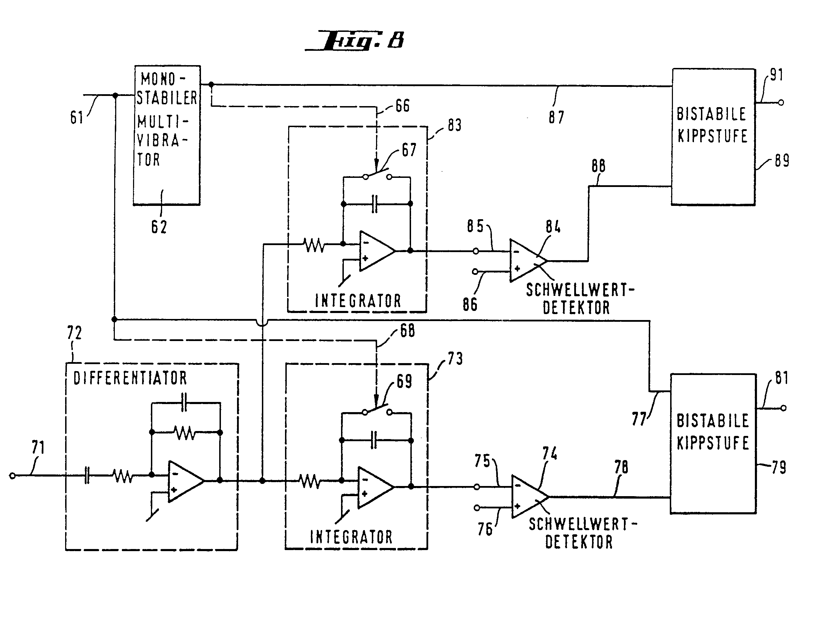

Die vorangehend beschriebene elektronische Schaltung ermöglicht die Durchführung der ersten Varianten des Verfahrens gemäß der Erfindung, die oben anhand von Fig. 1 erläutert wurde. Eine vollständigere Version der Einrichtung ist Fig. 8 dargestellt. Diese ermöglicht die Durchführung der bevorzugten Varianten des Verfahrens gemäß der Erfindung, die oben anhand von Fig. 3 und 4 erläutert wurde.

Die Fig. 8 dargestellte elektronische Einrichtung umfaßt außer den Elementen von Fig. 7 die folgenden weiteren Elemente:

- einen monostabilen Multivibrator, dessen Eingang über eine Leitung 61 Impulse zugeführt werden, die zugeordneten QRS-Komplexen entsprechen. Dieselben Impulse steuern (über eine durch die unterbrochene Linie 68 dargestellte Steuerung) das Schließen des Schalters 69 in dem Integrator 73. Sie werden außerdem dem Eingang 77 der Kippstufe 79 zugeführt;
- eine Integrator 83, der dieselbe Konfiguration und im Prinzip dieselbe Funktion hat wie der Integrator 73. Die Rücksetzung des Integrators 83 auf Null erfolgt durch kurzzeitiges Schließen eines Schalters 67. Dieses Schließen wird (über eine durch die unterbrochene Linie 66 dargestellte Steuerung) von den Impulsen veranlaßt, die am Ausgang des monostabilen Multivibrators 62 auftreten und die gegenüber den für die QRS-Komplexe repräsentativen Impulsen in bestimmter Weise, beispielsweise um 200 ms, verzögert sind;
- einen Schwellwertdetektor 84 mit einem Eingang, der über eine Leitung 85 mit dem Ausgang des Integrators 83 verbunden ist, und einem Eingang, dem über eine Leitung 86 eine Spannung zugeführt wird, die einen Schwellenwert darstellt. Das Ausgangsignal dieses Schwellwertdetektors wird an eine Leitung 88 abgegeben. Die Funktion des Schwellwertdetektors entspricht derjenigen des Schwellwertdetektors 74;
- eine bistabile D-Kippstufe 89, der als Eingangssignal einerseits über die Leitung 88 das Ausgangssignal des Schwellwertdetektors 84 und andererseits über eine Leitung 87 das Ausgangssignal des monostabilen Multivibrators 62 zugeführt wird. Das Ausgangssignal der Kippstufe 89 wird über eine Leitung 91 einem (in Fig. 8 nicht dargestellten) Analysator zugeführt. Dieser löst einen Atemstillstandsalarm nur dann aus, wenn die Ausgangssignale der Kippstufen 79 und 89 anzeigen, daß die Amplituden der Ausgangssignale der Integratoren 73 und 83 am Ende der betreffenden Integrationsintervalle den Schwellenwert während eines vorbestimmten Zeitintervalls nicht überschreiten.

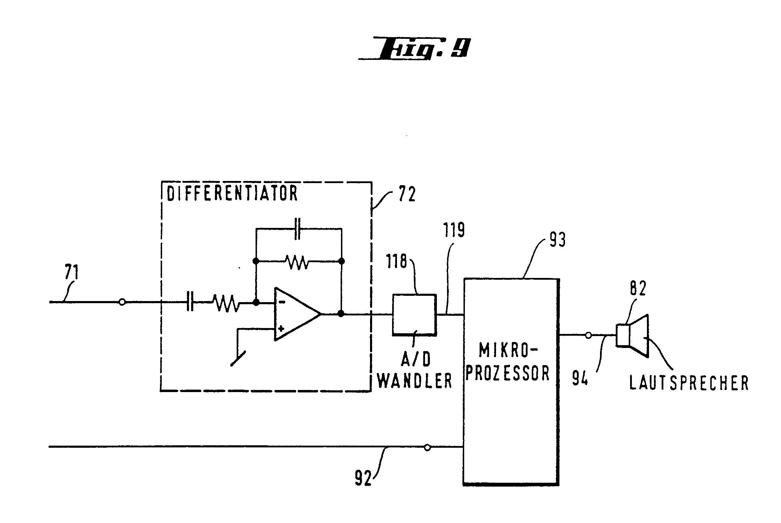

Fig. 9 zeigt eine zweite Ausführungsform der Einrichtungen gemäß Fig. 7 und 8. Bei der Schaltung gemäß Fig. 9 wurde die Differenzierschaltung 72 beibehalten, während die anderen in Fig. 7 und 8 dargestellten Elemente durch einen Mikroprozessor 93 ersetzt sind, der darüberhinaus die Funktion des anhand von Fig. 7 und 8 erwähnten (jedoch nicht dargestellten) Analysators ausübt. Zwischen den Ausgang der Differenzierschaltung 72 und einen Eingang 119 des Mikroprozessors 93 ist ein AnalogDigitalwandler 118 eingefügt. Impulse, die den QRS-Komplexen entsprechen und die auf einer Leitung 92 ankommen, werden an den "Interrupt"-Eingang des Mikroprozessors 93 angelegt. Dieser Mikroprozessor übt die Integrationsfunktion aus, indem er die Werte des Ausgangssignals der Differenzierschaltung 72, die während der Integrationsintervalle alle 1 ms gewonnen werden, kumuliert, wobei am Ende jedes dieser Intervalle auf Null zurückgesetzt wird. Der Mikroprozessor 93 vergleicht außerdem das Resultat dieser Integrationsvorgänge mit Detektionsschwellen und löst gegebenenfalls einen Alarm aus, der über einen Lautsprecher 82 hörbar gemacht wird, der durch ein auf einer Leitung 94 auftretendes Ausgangssignal des Mikroprozessors 93 aktiviert wird.

In Fig. 10 ist eine voll digitalisierte Version der Vorrichtung gemäß Fig. 9 dargestellt. In dieser Version führt ein Mikroprozessor 103 auch die Funktion der Differenzierschaltung 72 aus. Das auf der Leitung 71 ankommende Atmungssignal wird dem Eingang eines Analog-Digitalwandlers 118 zugeführt, dessen Ausgang mit dem Eingang 119 des Mikroprozessors verbunden ist.

Impulse, die den QRS-Komplexen entsprechen und über eine Leitung 102 ankommen, werden dem "Interrupt"-Eingang des Mikroprozessors zugeführt. Wie bei der Version gemäß Fig. 9 ist an den Ausgang 104 des Mikroprozessors ein Lautsprecher 82 angeschlossen, der die gegebenenfalls von dem Mikroprozessor ausgelösten Atemstillstandsalarme hörbar macht.

In dem Mikroprozessor 93 (Fig. 9) bzw. 103 (Fig. 10) wird alle 1 ms der Wert eines Signals berechnet, indem die Differenz zwischen zwei aufeinanderfolgenden Werten des Atmungssignals berechnet wird.

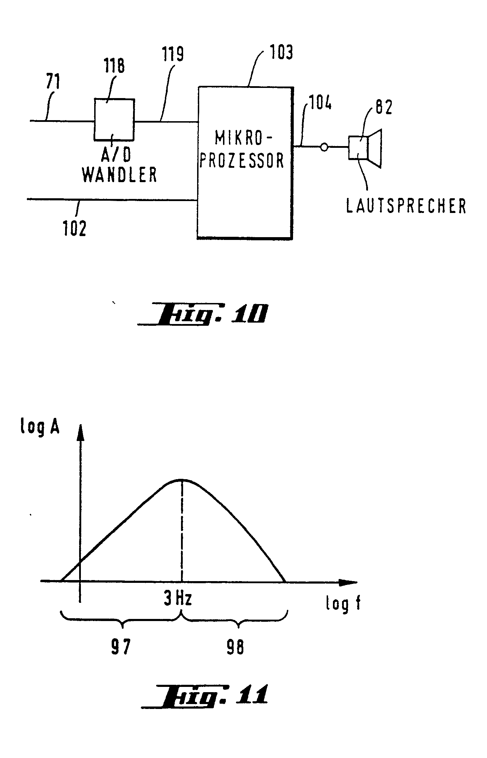

Fig. 11 zeigt eine typische Form der charakteristischen Amplituden-Frequenz-Kurve der Differenzierschaltung 72 in Fig. 7 bis 9 und 12. Die Mittenfrequenz von 3 Hz in Fig. 11 ist nur als Beispiel zu betrachten und keine notwendige Bedingung des Verfahrens.

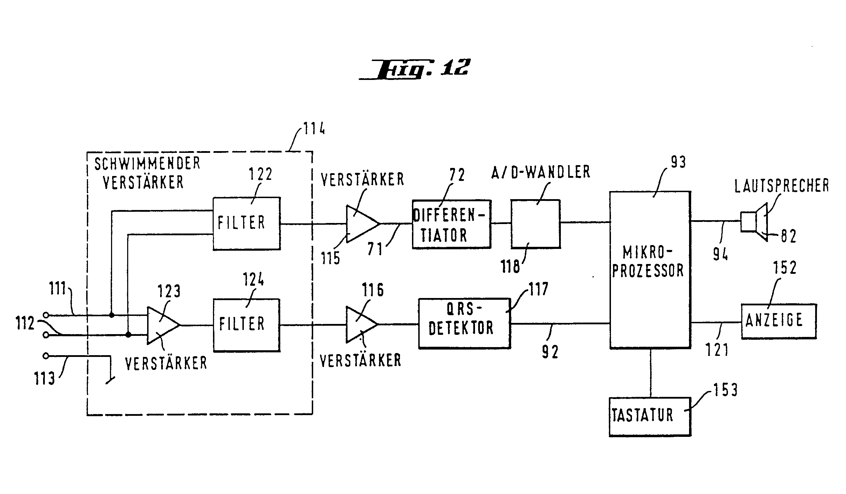

Fig. 12 zeigt das Blockschaltbild eines Gerät mit der Vorrichtung gemäß Fig. 9 für die Erfassung des Atemstillstands bei der Überwachung des Atmungssignals. Das in Fig. 12 dargestellte Gerät umfaßt zusätzlich zu den Elementen oben anhand von Fig. 9 beschriebenen Vorrichtung folgende Elemente:

- einen "schwimmenden" Verstärker 114, dessen Eingänge mit den in Fig. 1 dargestellten Elektroden 111, 112 und 113 verbunden sind. Einer der Ausgänge des Verstärkers 114 liefert ein Signal, das für die Atmung des Patienten kennzeichnend ist. Dieses Signal wird in einem Verstärker 115 verstärkt, und das verstärkte Signal wird dem Eingang der Differenzierschaltung 72 zugeführt;
- ein zweiter Ausgang des Verstärkers 114 liefert ein Signal, das für das Elektrokardiogramm des Patienten kennzeichnend ist. Dieses Signal wird in einem Verstärker 116 verstärkt, und das verstärkte Signal wird dem Eingang einer Schaltung 117 für die Detektion der QRS-Komplexe zugeführt. Die Schaltung

13

117 liefert an ihrem Ausgang Impulse, die für die genannten QRS-Komplexe kennzeichnend sind. Der Ausgang der Schaltung 117 ist über Leitung 92 mit dem "Interrupt"-Eingang des Mikroprozessors 93 verbunden;

- eine Tastatur 153 ist mit einem Eingang des Mikroprozessors 93 verbunden und dient dazu, diesem bestimmte Parameter zuzuführen, z. B. die Dauer des vorbestimmten Zeitintervalls, jenseits dessen ein Atemstillstandsalarm bei dem Verfahren gemäß der Erfindung ausgelöst wird;

- eine Vorrichtung 152 zur Anzeige numerischer Werte, z. B. eine Flüssigkeitskristallanzeige, ist über eine Leitung 121 mit einem Ausgang des Mikroprozessors 92 verbunden. Diese Vorrichtung ermöglicht die Anzeige der Alarmbedingungen, der Alarmursachen, des Inhalts des Arbeitsspeichers usw.

Fig. 12 zeigt ein Gerät, in dem das Atmungssignal und das Signal, das für das Elektrokardiogramm des Patienten kennzeichnend ist, mit demselben Elektrodensatz 111, 112 und 113 gewonnen werden. In diesem Fall umfaßt der schwimmende Verstärker 114 die Mittel, die für die Trennung des Atmungssignals von dem das Elektrokardiogramm repräsentierenden Signal erforderlich sind, z. B. ein Filter 122 für die Extraktion des Atmungssignals und die Serienschaltung eines Verstärkers 123 und eines Filters 124 für die Extraktion des das Elektrokardiogramm repräsentierenden Signals. Diese Signale werden an separate Ausgänge geliefert. Jedes dieser Signale kann indessen auch unabhängig von dem anderen, d. h. durch durch Einsatz separater Mittel, gewonnen werden.

In den oben beschriebenen Ausführungsbeispielen kann die Differenzierschaltung 72 durch Filtermittel ersetzt werden, die eine ähnliche Übertragungsfunktion haben.

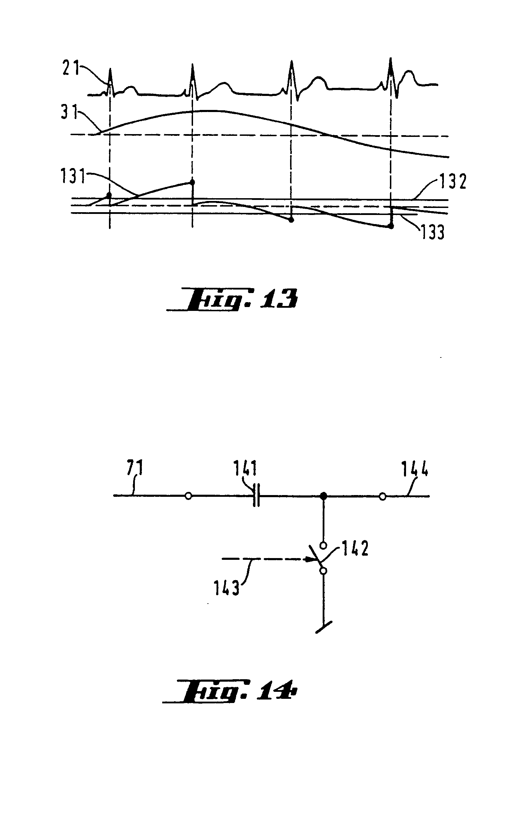

Fig. 13 zeigt eine zweite Ausführungsform des Verfahrens gemäß der Erfindung. In dieser Figur stellt eine unterbrochene horizontale Linie die Grundlinie jedes der gezeigten Signale dar. Das Auftreten der einzelnen QRS-Komplexe des Elektrokardiogramms ist jeweils durch eine unterbrochene vertikale Linie dargestellt. In dieser zweiten Ausführungsform wird das Atmungssignal jedesmal "geklemmt", wenn ein QRS-Komplex erfaßt wird. Man bildet auf diese Weise ein Signal 131, dessen Wellenform derjenigen des Atmungssignals in den Intervallen zwischen aufeinanderfolgenden QRS-Komplexen entspricht, das jedoch jedesmal den Wert Null annimmt, wenn ein QRS-Komplex festgestellt wird. Die Amplitudenwerte, die das Signal 131 in den Zeitpunkten annimmt, in denen die QRS-Komplexe auftreten, werden mit vorbestimmten Schwellenwerten 132, 133 verglichen. Falls die Amplituden des Signals 131 in den genannten Zeitpunkten die Schwellenwert 132 bzw. 133 während eines vorbestimmten Zeitintervalls nicht überschreiten, wird ein Alarm ausgelöst, der einen Atemstillstand anzeigt. In dem in Fig. 13 dargestellten ent-

14

gegengesetzten Fall, in dem die Amplituden des Signals 131 die genannten Schwellenwerte überschreiten, wird normale Atmung und mithin kein Atemstillstand konstatiert.

Fig. 14 zeigt in schematischer Darstellung eine Vorrichtung zur Durchführung des in Fig. 13 veranschaulichten Verfahrens. Diese Vorrichtung ermöglicht das "Klemmen" des auf der Leitung 71 gelieferten Atmungssignals auf analogem Wege. Die Vorrichtung umfaßt eine Verbindungskapazität 141 sowie einen Schalter 142. Um das Signal zu "klemmen", wird dieser Schalter beim Auftreten eines QRS-Komplexes kurzzeitig geschlossen. Das Schließen des Schalters 142 wird durch einen Impuls gesteuert, der für einen QRS-Komplex kennzeichnend ist. Diese Steuerung ist in Fig. 14 durch die unterbrochene Linie 142 angedeutet. Das klemmte Atmungssignal 131 wird über eine Leitung einer (in Fig. 14 nicht dargestellten) Analysierschaltung zugeführt, die einen Atemstillstandsalarm auslöst, wenn die Amplituden des Signals 131 während einer vorbestimmten Zeitspanne die Schwellenwerte 132, 133 nicht überschreiten. Die Funktion der in Fig. 14 dargestellten Vorrichtung läßt sich auch mit Hilfe eines Mikroprozessors realisieren.

**Patentansprüche**

1. Verfahren zur Überwachung von die Atmung eines Patienten kennzeichnenden Wellenformen und zur Detektion von Wellenformen mit Eigenschaften, die für einen Atemstillstand kennzeichnend sind, wobei das Verfahren die Verarbeitung eines ersten elektrischen Signals mit einer die Atmung des Patienten kennzeichnenden Wellenform und eines zweiten das Elektrokardiogramm des Patienten kennzeichnenden elektrischen Signals umfaßt, dadurch gekennzeichnet,

a) dass die Amplitude des ersten Signals, oder die Amplitude eines davon abgeleiteten Signals, im Zeitpunkt des Auftretens jedes QRS-Komplexes mit einem vorbestimmten Schwellenwert verglichen wird.

b) dass das erste Signal oder das davon abgeleitete Signal synchron mit dem Auftreten jedes QRS-Komplexes in dem zweiten Signal auf Null zurückgesetzt wird, und

c) dass ein einen Atemstillstand anzeigender Alarm ausgelöst wird, wenn die Amplituden des ersten Signals, oder des davon abgeleiteten Signals, in den genannten Zeitpunkten den Schwellenwert während eines vorbestimmten Zeitintervalls nicht überschreiten.

2. Verfahren nach Anspruch 1, dadurch gekennzeichnet,

a) daß ein drittes Signal gebildet wird, das für die erste zeitliche Ableitung des ersten Signals kennzeichnend ist,

b) daß ein viertes Signal gebildet wird, das für das Integral des dritten Signals über erste Integrationsintervalle kennzeichnend ist, wobei jedes dieser Integrationsintervalle der Zeitspanne zwischen zwei aufeinanderfolgenden QRS-Komplexen des zweiten Signals entspricht,

c) dass die Amplitude, die das vierte Signal im Zeitpunkt des Auftretens jedes das Ende eines der Integrationsintervalle markierenden QRS-Komplexen annimmt, mit einem vorbestimmten Schwellenwert verglichen wird,

d) dass das vierte Signal synchron mit dem Auftreten jedes QRS-Komplexes in dem zweiten Signal auf Null zurückgesetzt wird, und

e) daß ein einen Atemstillstand anzeigender Alarm ausgelöst wird, wenn die Amplituden des vierten Signals in den genannten Zeitpunkten den Schwellenwert während eines vorbestimmten Zeitintervalls nicht überschreiten.

3. Verfahren nach Anspruch 2, dadurch gekennzeichnet,

f) daß ein fünftes Signal gebildet wird, das für das Integral des dritten Signals über zweite Integrationsintervalle kennzeichnend ist, die jeweils eine vorbestimmte Zeitspanne gegenüber einem der ersten Integrationsintervalle versetzt sind,

g) dass die Amplitude des fünften Signals in dem das Ende jedes der zweiten Integrationsintervalle markierenden Zeitpunkt mit einem vorbestimmten Schwellenwert verglichen wird.

h) dass das fünfte Signal am Ende jedes zweiten Integrationsintervalls auf Null zurückgesetzt wird, und

i) daß ein einen Atemstillstand anzeigender Alarm ausgelöst wird, wenn die Amplituden des vierten Signals und die Amplituden des fünften Signals am Ende der entsprechenden Integrationsintervalle den genannten Schwellenwert während eines vorbestimmten Zeitintervalls nicht überschreiten.

4. Gerät zur Überwachung der Atmung eines Patienten mit Mitteln zur Bildung eines ersten elektrischen Signals mit einer Wellenform, die für die Atmung des Patienten kennzeichnend ist, und eines zweiten elektrischen Signals, das für das Elektrokardiogramm des Patienten kennzeichnend ist, gekennzeichnet durch

a) Mittel (72) zur Bildung eines dritten Signals, das für die erste zeitliche Ableitung des ersten Signal kennzeichnend ist,

b) Mittel (73) zur Bildung eines vierten Signals, das für das Integral des dritten Signals über erste Integrationsintervalle kennzeichnend ist, wobei jedes dieser Integrationsintervalle dem Intervall zwischen zwei aufeinanderfolgenden QRS-Komplexen in dem zweiten Signal entspricht,

c) Mittel (74) zum Vergleichen der Amplitude des vierten Signals in dem das Ende eines der genannten Integrationsintervalle markierenden Zeitpunkt des Auftretens jedes QRS-Komplexes mit einem vorbestimmten Schwellenwert.

d) Mittel (68, 69) zur Rücksetzung des vierten Signals auf Null synchron mit dem Auftreten jedes QRS-Komplexes in dem zweiten Signal, sowie

e) Mittel (93) zur Auslösung eines einen Atemstillstand anzeigenden Alarms, wenn die Amplituden des vierten Signals i den genannten Zeitpunkten den Schwellenwert während eines vor bestimmten Zeitintervalls nicht überschreiten.

5. Gerät nach Anspruch 4, gekennzeichnet durch

f) Mittel (62, 83) zur Bildung eines fünften Signals, das für das Integral des dritten Signals über zweite Integrations intervalle kennzeichnend ist, wobei jedes dieser zweiten Integrationsintervalle um eine vorbestimmte Zeitspanne gegenüber einem der ersten Integrationsintervalle versetzt ist,

g) Mittel (74) zum Vergleichen der Amplitude des fünften Signals in dem das Ende jedes der zweiten Integrationsintervalle markierenden Zeitpunkt mit einem vorbestimmten Schwellenwert,

h) Mittel (66, 67) zur Rücksetzung des fünften Signals auf Null am Ende jedes der zweiten Integrationsintervalle, sowie ferner dadurch gekennzeichnet,

i) daß die Mittel (79, 89) zur Auslösung des Alarms diesen nur dann auslösen, wenn die Amplituden des vierten Signals und die Amplituden des fünften Signals am Ende der entsprechenden Integrationsintervalle den Schwellenwert während eines vorbestimmten Zeitintervalls nicht überschreiten.

6. Gerät nach Anspruch 4, dadurch gekennzeichnet, daß die Mittel zur Bildung des dritten Signals Filtermittel sind, deren Dämpfungs-Frequenz-Kurve derjenigen einer Differenzier schaltung gleicht.

7. Gerät nach Anspruch 5, dadurch gekennzeichnet, dass die Mittel zur Bildung des dritten Signals Filtermittel sind, deren Dämpfungs-Frequenz-Kurve derjenigen einer Differenzierschaltung gleicht.

8. Gerät zur Überwachung der Atmung eines Patienten mit Mitteln zur Bildung eines ersten elektrischen Signal mit einer Wellenform, die für die Atmung des Patienten kennzeichnend ist, und eines zweiten elektrischen Signals, das für das Elektrokardiogramm des Patienten kennzeichnend ist, gekennzeichnet durch

a) Mittel zum Vergleichen der Amplitude des ersten Signals im Zeitpunkt des Auftretens je-

des QRS-Komplexes mit einem vorbestimmten Schwellenwert.

b) Mittel (143, 142) zur Rücksetzung des ersten Signals auf Null synchron mit dem Auftreten jedes QRS-Komplexes in dem zweiten Signal, sowie

c) Mittel zur Auslösung eines einen Atemstillstand anzeigenden Alarms, wenn die Amplituden des ersten Signals in den genannten Zeitpunkten den Schwellenwert während eines vorbestimmten Zeitintervalls nicht überschreiten.

## Claims

1. A method of monitoring waveforms representing the respiration of a patient, and of detecting waveforms having characteristics indicative of apnea, said method comprising processing a first electrical signal having a waveform representing the respiration of the patient and a second electrical signal representing the electrocardiogram of the patient, said method being characterised in that

a) the amplitude of the first signal or the amplitude of a signal derived therefrom is compared with a predetermined threshold value at the time of incidence of each QRS complex,

b) The first signal or the signal derived therefrom is reset to zero in synchronism with the incidence of each QRS complex in the second signal,

c) an apnea alarm is triggered if at the said times the amplitudes of the first signal or of the signal derived therefrom do not exceed the threshold value over a predetermined interval of time.

2. A method according to claim 1, characterised in that

a) a third signal is formed which represents the first derivative of the first signal with respect to time,

b) a fourth signal is formed which represents the integral of the third signal over first integration intervals, each of these intervals corresponding to the interval between two successive QRS complexes of the second signal,

c) the amplitude of the fourth signal at the time of the incidence of each QRS complex which marks the end of one of the said integration intervals is compared with a predetermined threshold value,

d) the fourth signal is reset to zero in synchronism with the incidence of each QRS complex in the second signal, and

e) an apnea alarm is triggered if the amplitudes of the fourth signal at the said times do not exceed the threshold value over a predetermined interval of time.

3. A method according to claim 2, characterised in that

f) a fifth signal is formed which represents the integral of the third signal over second integration intervals, each of which is offset by a predetermined interval of time with respect to one of the first integration intervals,

g) the amplitude of the fifth signal at the time marking the end of each of the second integration intervals is compared with a predetermined threshold value and

h) the fifth signal is reset to zero at the end of each second integration interval,

i) an apnea alarm is triggered if the amplitudes of the fourth signal and the amplitudes of the fifth signal at the end of the respective integration intervals do not exceed the threshold value over a predetermined interval of time.

4. Apparatus for monitoring the respiratiorn of a patient, comprising means for forming a first electrical signal having a waveform representing the respiration of the patient and a second electrical signal representing the electrocardiogram of the patient, characterised by:

a) means (72) for forming a third signal representing the first derivative of the first signal with respect to time,

b) means (73) for forming a fourth signal representing the integral of the third signal over first integration intervals, each of said intervals corresponding to the interval between two successive QRS complexes in the second signal,

c) means (74) for comparing the amplitude of the fourth signal at the time of incidence of each QRS complex which marks the end of one of said integration intervals with a predetermined threshold value,

d) means (68, 69) for resetting the fourth signal to zero in synchronism with the incidence of each QRS complex in the second signal and

e) means (93) for triggering an apnea alarm if the amplitudes of the fourth signal at said times do not exceed the threshold value over a predetermined interval of time.

5. Apparatus according to claim 4, characterised by:

f) means (62, 83) for forming a fifth signal representing the integral of the third signal over second integration intervals, each of which is offset by a predetermined interval of time with respect to one of the first integration intervals,

g) means, (74) fur comparing the amplitude of the fifth signal at the time marking the end of each of the second integration intervals with a predetermined threshold value,

h) means (66, 67) for resetting the fifth signal to zero at the end of each of the second integration intervals,

and further characterised in that

i) the means (79, 89) for triggering the alarm trigger the latter only if the amplitudes of the fourth signal and the amplitudes of the fifth signal at the end of the respective integration intervals do not exceed the threshold value over a predetermined interval of time.

6. Apparatus according to claim 4, characterised in that the means for forming the third signal are filter means having a frequency attenuation characteristic similar to that of a differentiating circuit.

7. Apparatus according to claim 5, characterised in that the means for forming the third signal are filter means having a frequency attenuation, characteristic similar to that of a differentiating circuit.

8. Apparatus for monitoring the respiration of a patient, comprising means fur forming a first electrical signal having a waveform representing the respiration of the patient and a second electrical signal representing the electrocardiogram of the patient, characterised by:

a) means for comparing the amplitude of the first signal at the time of incidence of each QRS complex with a predetermined threshold value,

b) means (143, 142) for resetting the first signal to zero in synchronism with the incidence of each QRS complex in the second signal, and

c) means for triggering an apnea alarm if the amplitudes of the first signal at the said times do not exceed the threshold value over a predetermined interval of time.

**Revendications**

1. Procédé pour la surveillance de formes d'ondes représentatives de la respiration d'un patient et pour la détection de formes d'onde présentant des caractéristiques indicatives d'une apnée, ce procédé comprenant le traitement d'un premier signal électrique ayant une forme d'onde représentative de la respiration du patient et un deuxième signal électrique représentatif de l'électrocardiogramme du patient.

ledit procédé étant caractérisé en ce que

a) l'amplitude prise par le premier signal, ou l'amplitude prise par un signal obtenu par transformation du premier signal, à l'instant de l'avènement de chaque complexe QRS est comparée avec une valeur de seuil prédéterminée,

b) le premier signal ou le signal obtenu par transformation de celui-ci est remis à zéro en synchronisme avec l'avènement de chaque complexe QRS sur le deuxième signal, et

c) une alarme est déclenchée si les amplitudes du premier signal, ou du signal obtenu par transformation de celui-ci, auxdits instants ne dépassent pas la valeur de seuil sur un intervalle de temps prédéterminé, cette alarme étant indicative d'une apnée.

2. Procédé selon la revendication 1, caractérisé en ce que

a) l'on forme un troisième signal représentatif de la dérivée première du premier signal par rapport au temps,

b) l'on forme un quatrième signal représentatif de l'intégrale du troisième signal sur des premiers intervalles d'intégration, chacun de ces intervalles correspondant à l'intervalle entre deux complexes QRS successifs du deuxième signal,

c) l'on compare l'amplitude prise par le quatrième signal à l'instant de l'avènement de chaque complexe QRS qui marque la fin d'un desdits intervalles d'intégration avec une valeur de seuil prédéterminée,

d) l'on remet le quatrième signal à zéro en synchronisme avec l'avènement de chaque complexe QRS sur le deuxième signal, et

e) l'on déclenche une alarme si les amplitudes prises par le quatrième signal auxdits instants ne dépassent pas la valeur de seuil sur un intervalle de temps prédéterminé, cette alarme étant indicative d'une apnée.

3. Procédé selon la revendication 2, caractérisé en ce que

f) l'on forme un cinquième signal représentatif de l'intégrale du troisième signal sur des deuxièmes intervalles d'intégration, chacun desquels est décalé d'un intervalle de temps prédéterminé par rapport à un des premiers intervalles d'intégration,

g) l'on compare l'amplitude prise par le cinquième signal à l'instant qui marque la fin de chaque deuxième intervalle d'intégration avec une valeur de seuil prédéterminée,

h) l'on remet le cinquième signal à zéro à la fin de chaque deuxième intervalle d'intégration, et

i) l'on déclenche l'alarme seulement si les amplitudes prises par le quatrième signal et les amplitudes prises par le cinquième signal à la fin des intervalles d'intégration respectifs ne dépassent pas la valeur de seuil sur un intervalle de temps prédéterminé.

4. Appareil pour la surveillance de la respiration d'un patient comportant des moyens pour former un premier signal électrique ayant une forme d'onde représentative de la respiration du patient et un deuxième signal électrique représentatif de l'électrocardiogramme du patient,

ledit appareil étant caractérisé en ce qu'il comporte

a) des moyens (72) pour former un troisième signal représentatif de la dérivée première du premier signal par rapport au temps,

b) des moyens (73) pour former un quatrième signal représentatif de l'intégrale du troisième signal sur des premiers intervalles d'intégration, chacun de ces intervalles correspondant à l'intervalle entre deux complexes QRS successifs du deuxième signal,

c) des moyens (74) pour comparer l'amplitude prise par le quatrième signal à l'instant de l'avènement de chaque complexe QRS qui marque la fin d'un desdits intervalles d'intégration avec une valeur de seuil prédéterminée,

d) des moyens (68, 69) pour remettre le quatrième signal à zéro en synchronisme avec l'avènement de chaque complexe QRS sur le deuxième signal, et

e) des moyens (93) pour déclencher une alarme si les amplitudes du quatrième signal auxdits instants ne dépassent pas la valeur de seuil sur un intervalle de temps prédéterminé, cette alarme étant indicative d'une apnée.

5. Appareil selon la revendication 4, caractérisé en ce qu'il comporte

f) des moyens (62, 83) pour former un cinquième signal représentatif de l'intégrale du troisième signal sur des deuxièmes intervalles d'intégration, chacun desquels est décalé d'un intervalle de temps prédéterminé par rapport à un des premiers intervalles d'intégration,

g) des moyens (84) pour comparer l'amplitude prise par le cinquième signal à l'instant qui marque la fin de chaque deuxième intervalle d'intégration avec une valeur de seuil prédéterminée,

h) des moyens (66, 67) pour remettre le cinquième signal à zéro à la fin de chaque deuxième intervalle d'intégration,

et en ce que

i) les moyens (79, 89) pour déclencher l'alarme ne déclenchent celle-ci que si les amplitudes prises par le quatrième signal et les amplitudes prises par le cinquième signal à la fin des intervalles d'intégration respectifs ne dépassent pas la valeur de seuil sur un intervalle de temps prédéterminé.

6. Appareil selon la revendication 4 caractérisé en ce que les moyens pour former le troisième signal sont des moyens de filtrage ayant une caractéristique atténuation-fréquence similaire à celle d'un circuit dérivateur.

7. Appareil selon la revendication 5, caractérisé en ce que les moyens pour former le troisième signal sont des moyens de filtrage ayant une caractéristique atténuation-fréquence similaire à celle d'un circuit dérivateur.

8. Appareil pour la surveillance de la respiration d'un patient comportant des moyens pour former un premier signal électrique ayant une forme d'onde représentative de la respiration du patient et un deuxième signal électrique représentatif de l'électrocardiogramme du patient,

ledit appareil étant caractérisé en ce qu'il comporte

a) des moyens pour comparer l'amplitude prise par le premier signal à l'instant de l'avènement de chaque complexe QRS avec une valeur de seuil prédéterminée,

b) des moyens (143, 142) pour remettre le premier signal à zéro en synchronisme avec l'avènement de chaque complexe QRS sur le deuxième signal, et

c) des moyens pour déclencher une alarme si les amplitudes du premier signal auxdits instants ne dépassent pas la valeur de seuil sur un intervalle de temps prédéterminé, cette alarme étant indicative d'une apnée.

_Fig. 1_

1

EP 0 212 370 B1

# Fig. 2

**Fig. 3**

**Fig. 4**

_Fig. 5_

## Fig. 6

*Fig. 7*

Fig. 8

15

Fig. 9

*Fig. 10*

*Fig. 11*

## _Fig. 12_

21

31

131

132

133

**_Fig. 13_**

71   141   144

142

143

**_Fig. 14_**